# EUROPEAN PATENT SPECIFICATION

(11) **EP 1 504 265 B1**
(45) Date of publication and mention of the grant of the patent: **30.11.2011**
(21) Application number: 03723579.3
(22) Date of filing: 29.04.2003
(51) Int. Cl.: G01N 33/543, G01N 33/68

(54) **SANDWICH ASSAY**
SANDWICH-ASSAY
DOSAGE EN SANDWICH

(30) Priority: 29.04.2002 SE 0201306; 29.04.2002 US 375823 P
(43) Date of publication of application: 09.02.2005
(73) Proprietor: Affibody AB, 161 02 Bromma (SE)
(72) Inventor: AHLBORG, Niklas, S-116 61 Stockholm (SE); NYGREN, Per-Ake, S-178 40 Ekerö (SE)
(74) Representative: Mattsson, Niklas
(86) International application number: PCT/SE2003/000703
(87) International publication number: WO 2003/093821

(56) References cited:
- WO-A1-00/63243
- WO-A2-01/81924
- US-A- 5 801 064
- US-A- 6 107 045
- DATABASE MEDLINE [Online] MOYLE W.R. ET AL.: 'Bioimmunoassay (BIA): a sandwich immunoassay scheme employing monoclonal antibodies and hormone receptors to quantify analytes', XP002966422 Retrieved from NCBI Database accession no. 88259064 & J. RECEPT. RES. vol. 8, no. 1-4, 1988, pages 419 - 436
- HANSSON M. ET AL.: 'An in vitro selected binding protein (affibody) shows conformation-dependent recognition of the respiratory syncytial virus (RSV) G protein' IMMUNOTECHNOLOGY vol. 4, 1999, pages 237 - 252, XP004163551
- NORD KARIN ET AL.: 'Binding proteins selected from combinatorial libraries of an alpha-helical bacterial receptor domain' NATURE BIOTECHNOLOGY vol. 15, August 1997, pages 772 - 777, XP002923927
- GUNNERIUSSON ELIN ET AL.: 'Staphylococcal surface display of immunoglobulin A (IgA)- and IgE-specific in vitro-selected binding proteins (affibodies) based on staphylococcus aureus protein A' APPLIED AND ENVIRONMENTAL MICROBIOLOGY vol. 65, no. 9, September 1999, pages 4134 - 4140, XP002966423

## Description

### Field of the invention

The present invention relates to a sandwich assay method for the detection of a target molecule in a sample comprising a complex biological fluid.

### Background

Antibody-based capture immunoassays (e g sandwich immunoassays) are used to detect and quantify a variety of molecules, including immunoglobulins (Ig), hormones, cytokines, complement factors and other molecules found in biological samples, such as human serum or plasma. Briefly, in sandwich immunoassays, a first, "capture", antibody having affinity for the intended target is immobilized to a solid support, and subsequently exposed to a sample. Target molecules present in the sample bind to the capture antibody, and excess sample is removed. The bound target molecules are exposed to a second, "detector", antibody having affinity for the target molecule. After removal of any excess detector antibody, the presence of detector antibody bound to the complex between target and capture antibody is measured using one or more of a variety of known detecting principles. A positive signal is obtained when a detector antibody has bound to a target molecule bound to the capture antibody.

In particular, the use of capture ELISA (Enzyme-Linked ImmunoSorbent Assay) for this purpose is widely spread, which may be explained by the generally high specificity and sensitivity of the assay. A capture immunoassay can be used to quantify virtually any soluble and antigenic molecule having a size sufficient to harbor the binding of a capture antibody and a detection antibody simultaneously. When used in combination with an amplification system, e g biotinylated detection antibodies and avidin-enzyme conjugates, the sensitivity of such assays may reach a lower detection limit at the picogram/ml level, e g in the case of protein antigens.

However, the use of present capture immunoassays for the detection and quantification of molecules in complex biological fluids, e g human serum or any other biological fluid comprising antibodies, presents problems despite their general usefulness. Thus, it is well known in the art that such complex biological fluids may contain antibodies that are reactive with respect to the antibodies used in immunoassays, giving rise to false positive signals through the cross-linking of capture and detector antibody without the presence of a target molecule. Human samples may contain heterophilic anti-animal Ig antibodies (HAIA) that interfere with the analysis of target molecules in the sample through such cross-linking (Reinsberg, Clin Biochem 29:145 (1996); Kricka, Clin Chem 45:942 (1999); Klee, Arch Pathol Lab Med 124:921 (2000); Kellar et al, Cytometry 45:27 (2001)). HAIA were found in 27-40% of serum from healthy individuals (Boscato and Stuart, Clin Chem 34:27 (1988); Hennig et al, J Immunol Methods 235:71 (2000)), and even higher prevalences have been reported (Kricka, *supra).* A large number of studies have described false positive signals caused by heterophilic antibodies in immunoassays, including commercial assays used for diagnostic purposes (Hennig et al, *supra;* Ingels et al, Clin Toxicol 38:343 (2000); Kellar *et al, supra*).

Because of the problems described above, arising from the use of immunoassays in the analysis of complex biological fluid samples comprising antibodies, several strategies have been explored in order to reduce the interference caused by such antibodies. Thus, addition of excessive irrelevant animal immunoglobulins, removal of human antibodies from human serum, neutralization of HAIA reactivity by addition of e g mouse anti-human IgM antibodies, use of fragmented antibodies (Fab or F(ab')₂) and use of combinations of antibodies from evolutionary distant species have all been investigated (Reinsberg, supra; Kricka, *supra;* Span et al, Int J Biol Markers 15:184 (2000); Butler and Cole, Clin Chem 47:1332 (2001)). These methods may be efficient in preventing interference but can falter, thus necessitating additional controls to ensure that interference really is circumvented as intended (Reinsberg, *supra*; Kricka, *supra).* For example, the use of Fab fragments may bypass the risk of interference of Fc-reactive HAIA in a human serum sample. However, since HAIA reactivity with the Fab region has also been identified, this approach is unlikely to be sufficient (Hennig et *al, supra*). The economical aspect should also be taken into account, since inclusion of additional antibody reagents, other than those used for capture and detection, may actually contribute significantly to the assay cost. By using combinations of avian and mammalian antibodies the risk of HAIA interference can be drastically reduced (Span *et al, supra*), but that strategy does not permit development of assays with mono-specific reagents.

Thus, there is a need for novel assay methods which obviate problems that have been described above in relation to the use of known immunoassay methods in the analysis of samples comprising a complex biological fluid.

### Summary of the invention

It is an object of the present invention to meet this need through the provision of an improved method for sandwich detection of target molecules using affinity ligands.

Another object of the invention is to provide a sandwich assay method which is less sensitive to interference from cross-linking antibodies than prior art methods.

Yet another object of the invention is to enable the use of combinations of affinity ligands with different properties in a novel sandwich assay method, in order to optimize the performance of the assay in terms of sensitivity, reduction of interference, robustness or any other parameter of interest.

The foregoing and other objects, apparent to the skilled man from the present disclosure, are met by the invention as claimed. Thus, a sandwich assay method for detecting the presence of a target molecule in a human sample comprising a complex biological fluid containing heterophilic anti-animal Ig antibodies is provided. The assay method comprises:
- providing a first affinity ligand with affinity for the target molecule, which affinity ligand is capable of being immobilized to a solid support;
- applying the sample in such a way that binding of a target molecule, if present in the sample, to the first affinity ligand is enabled;
- applying a second affinity ligand with affinity for the target molecule, the application enabling binding of the second affinity ligand to the target molecule;
- removing second affinity ligand not bound to target molecule; and
- detecting the presence of the second affinity ligand, such presence being an indicator of the presence of a target molecule in the sample;

the first affinity ligand being immobilized to the solid support at any stage before said detection,
in which method at least one of the first and second affinity ligands is an affinity ligand other than an antibody as defined in claim 1.

Thus, the present invention is based on the finding that replacement of one or more antibodies by non-antibody affinity ligands in a sandwich assay alleviates or solves the problems with false positive signals in the analysis of samples comprising complex biological fluids. The primary function of the method of the invention is to reduce cross-linking of the first and second affinity ligands by antibodies present in the sample. This is further achieved through the provision of first and second affinity ligands that are different from each other in terms of structure or origin. However, the invention also makes possible the use of first and second non-antibody affinity ligands that are both of the same type. The problem of cross-linking is then alleviated by the fact that the type of affinity ligand used in the method is not as easily cross-linked by antibodies as the "capture" and "detector" antibodies of prior art sandwich assays, or by the fact that much fewer antibodies able to crosslink these ligands exist in samples. Thus, in the method of the invention, one of the first and second affinity ligands may well be an antibody, but also contemplated is use of affinity ligands other than antibodies as both "capture" and "detector" ligands.

The method of the invention is suitable for the analysis of samples with any concentration of target molecules. However, the advantages of the invention are particularly evident in the case of detection of very low concentrations of target molecule in a sample, i e when the sample may only be diluted to a small extent when carrying out the assay. This is because the problem of cross-linking in samples comprising a complex biological fluid is particularly troublesome in the analysis of complex biological fluids at high concentrations, e g that have not been diluted. Whereas methods of the prior art exhibit interference problems in the analysis of undiluted, or little diluted, samples of complex biological fluids, the method of the invention enables the accurate measuring of target molecule also in such samples, making possible detection of lower concentrations of target molecule. Thus, for example, analysis of rare target molecules in samples of high serum concentrations is made possible through the present invention.

### Brief description of the drawings

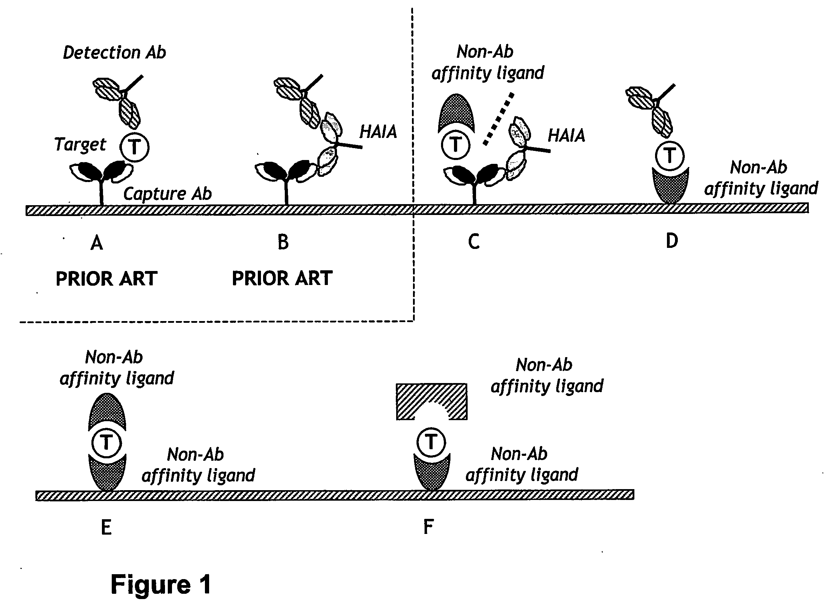

Figure 1: schematic illustration of the prior art in comparison with the invention, as well as of different embodiments of the invention. A: prior art detection of target molecule by two antibodies; B: prior art cross-linking by HAIA between capture and detector antibodies; C: a non-antibody affinity ligand as second affinity ligand in accordance with the invention; D: a non-antibody affinity ligand as first affinity ligand in accordance with the invention; E: two non-antibody affinity ligands of the same type as first and second affinity ligands in accordance with the invention; and F: two non-antibody affinity ligands of different types as first and second affinity ligands in accordance with the invention.

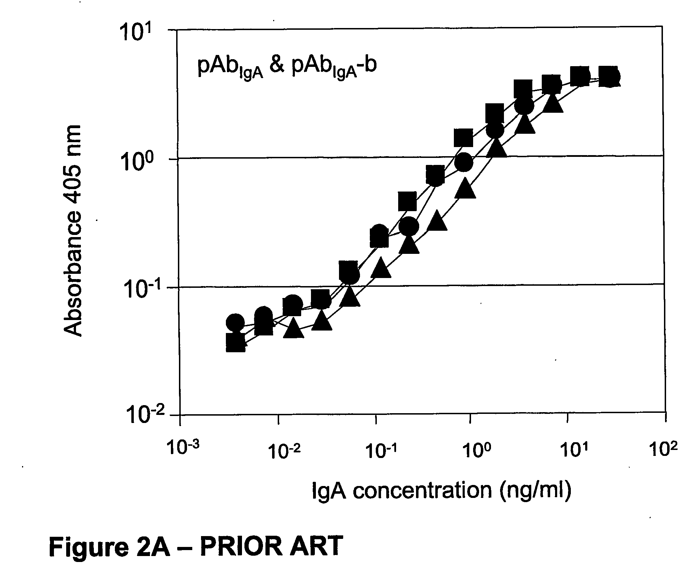

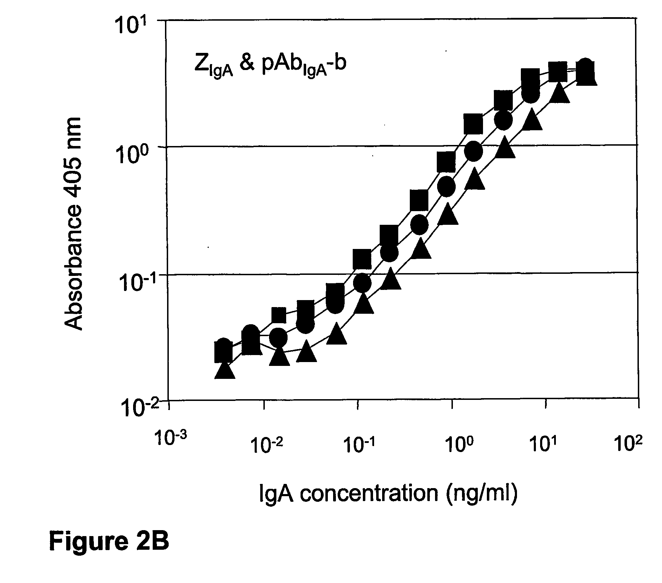

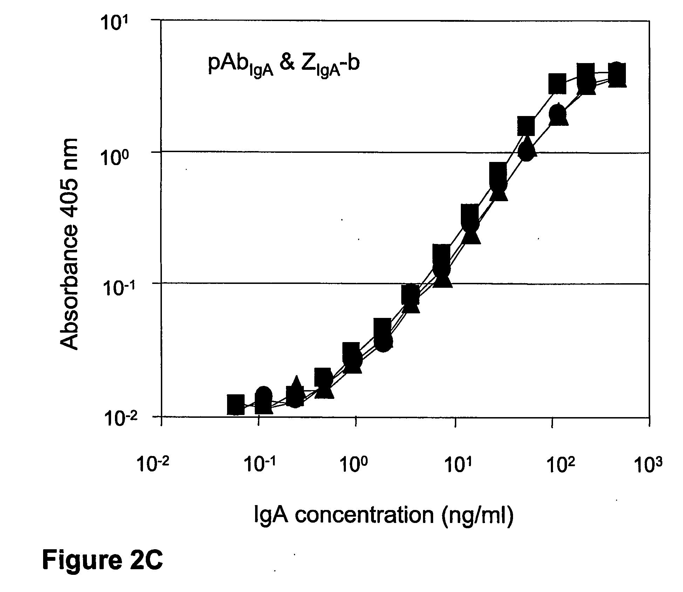

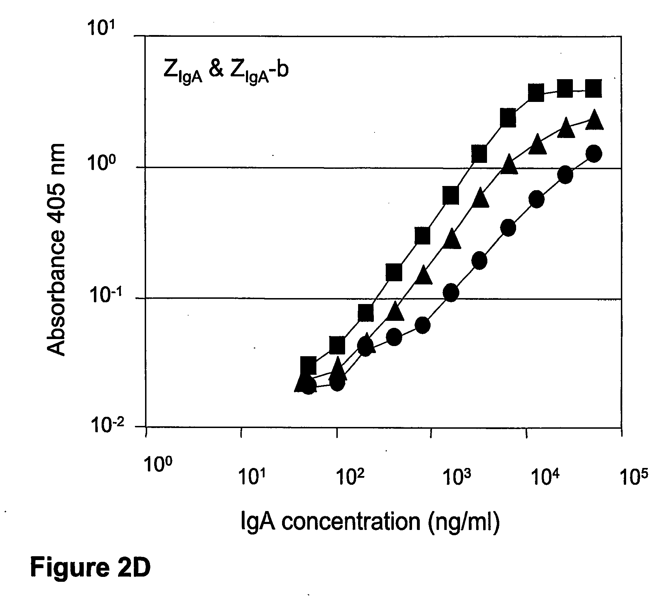

Figure 2: Sandwich assay analysis of IgA using an Affibody® (Z_{IgA}) and/or polyclonal antibodies (pAb_{IgA}). Two-fold serial dilutions of IgA1, IgA2 and IgA1/IgA2 standard were analyzed using combinations of Z_{IgA} and/or pAb_{IgA} as indicated. The symbols display reactivity with IgA1/IgA2 (circles), IgA1 (squares) and IgA2 (triangles) standard. Note the different concentration ranges of IgA displayed in the graphs.

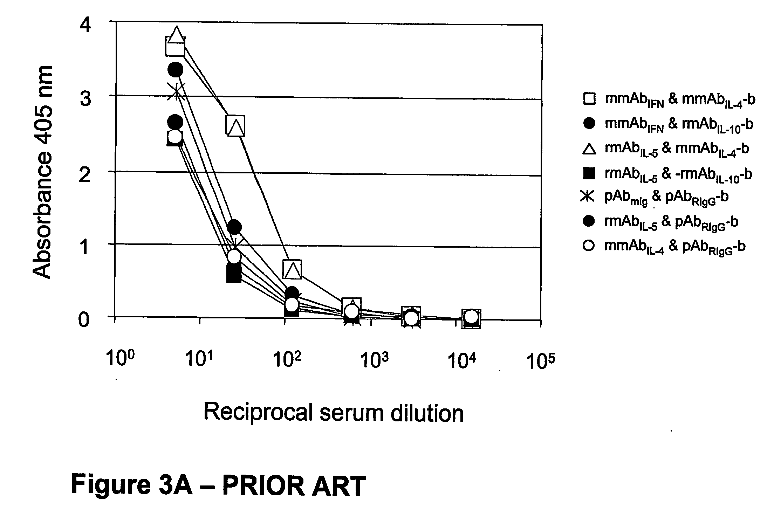

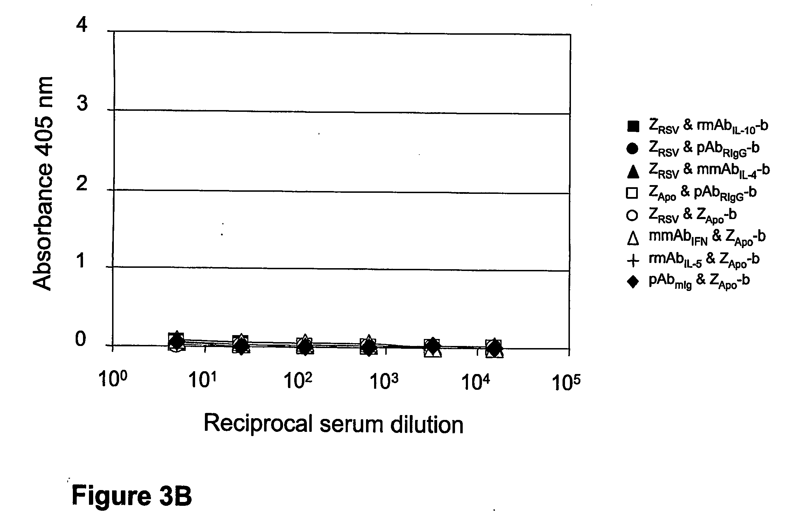

Figure 3: Human serum antibody interference in sandwich assay systems based on antibodies and/or Affibody® molecules displaying non-matched specificities. Capture reagents were incubated with serum at consecutive fivefold dilutions ranging from 5 to 15,625 and the binding of detection reagent was measured. The graphs display combinations of antibodies (A) (according to the prior art) or combinations of Affibody® molecules with either antibodies or Affibody® molecules (B) (according to the invention). The symbols indicate combinations of reagents as described in Table 1.

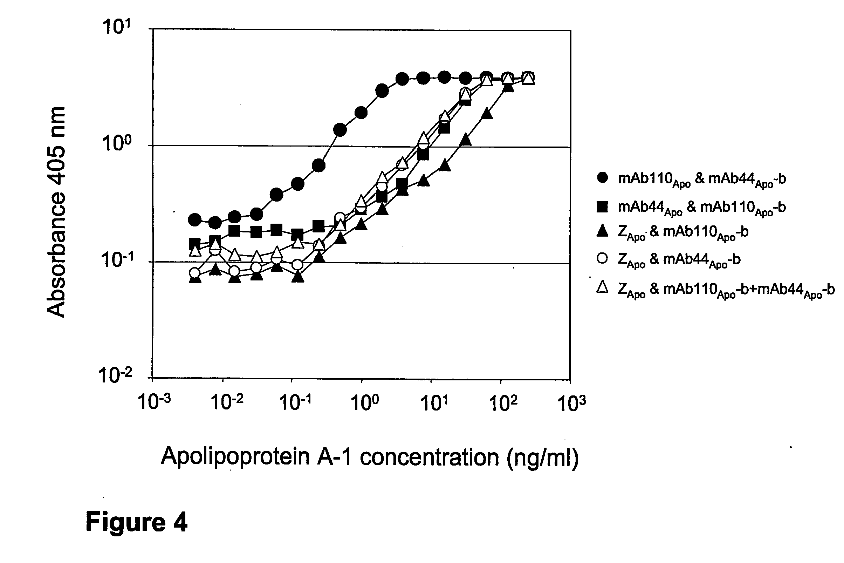

Figure 4: Comparison of sandwich assay systems for apolipoprotein A-1 using Z_{Apo} and/or specific mAbs. All possible criss-cross combinations of Z_{Apo}, mAb110_{Apo} and mAb44_{Apo} were used to measure apolipoprotein A-1 standard. Shown are all combinations that detected apolipoprotein A-1 at concentrations ranging from 4 pg/ml up to 250 ng/ml.

### Detailed description of the invention

The invention relates to a sandwich assay method for detection of a target molecule in a sample comprising a complex biological fluid. The target molecule may be any soluble substance or particle (e g virus particle) for which it is possible to obtain affinity ligands. For example, in the case of antibody affinity ligands obtained by induction of an antibody response to the target molecule, this means that the target molecule has to be immunogenic. As another example, in the case of affinity ligands of both immunoglobulin and non-immunoglobulin protein classes obtained by selection from protein libraries by for example phage-display selection technology or other *in vitro* selection systems, the target molecule must be compatible with such a selection system. Non-limiting examples of possible target molecules are immunoglobulins, hormones, cytokines, complement factors, other serum proteins, enzymes, small molecules, pharmaceuticals, molecules from infectious agents such as bacterial, viral and parasite antigens, tumor antigens, peptides and nucleic acids. In general, any target molecule susceptible for detection with a traditional ELISA or other known sandwich assay is contemplated in the context of the present invention.

The sample that is to be tested for presence of the target molecule comprises a complex biological fluid. In the context of the invention, such a complex biological fluid is defined as one normally associated with the above-mentioned problems with false positives in traditional immunoassays, and thus one that is likely to comprise antibodies of various specificities. Commonly, samples for use in the present invention are drawn from human or animal subjects, and may for example comprise complex biological fluids such as serum, plasma, saliva, whole blood, plasma from plasmapheresis, cerebrospinal fluid, amniotic fluid, urine, semen, cord blood, supernatants from cell culture, cell culture media, exudate or aspirate.

The sandwich assay method of the invention comprises a number of steps, which will in the following be set out in their preferred order of performance. Although some of the steps plainly need to be performed in the order described, this order of performance is not intended as limiting. For example, the removal of excess reagents may be performed at several stages during the execution of the method. Another example regards the immobilization of the first affinity ligand to a solid support, which for example may be performed prior to carrying out the method of the invention, or performed subsequent to the formation of a complex between the affinity ligands and the target molecule. The person of skill in the art of traditional sandwich immunoassays will be able to establish the preferred protocol in each case without undue experimentation.

The method of the present invention is carried out in the context of a solid support, to which the first affinity ligand is capable of being immobilized. The solid support determines the format of the analysis, and may be chosen freely among suitable such supports. For example, the microtiter plates used as standard for known immunoassays may advantageously also be used in the method of the invention. Microtiter plates for use as support may for reasons of commercial availability have 48, 96 or 384 wells. Examples of other suitable solid supports, on which the sandwich assay method of the invention may be performed, are compact discs comprising microfluidic channel structures; protein array chips; membranes, e g nitrocellulose or PVDF membranes; microparticles, e g paramagnetic or non-paramagnetic beads; pin structures; stick structures, e g dip sticks; sensor surfaces for biosensors, e g sensor chip surfaces for use in surface plasmon resonance instruments; and cell surfaces. Immobilization of the first affinity ligand to such solid supports or substrates may be performed using covalent or non-covalent coupling procedures available for the substrate chosen, and is well within the grasp of the skilled person.

The method of the present invention comprises the step of providing a first affinity ligand with affinity for the target molecule, which affinity ligand is capable of being immobilized to a solid support. In an embodiment of the invention, the first affinity ligand is immobilized to a solid support prior to carrying out of the assay method. For example, the first affinity ligand may be immobilized to the inside of at least one of the wells of a microtiter plate, or to any other of the solid support structures mentioned above. However, it is also contemplated that the first affinity ligand be provided in solution. In such a case, the method of the invention comprises immobilizing the first affinity ligand to a solid support during the course of the method, e g subsequent to formation of a tertiary complex between first affinity ligand, target molecule and second affinity ligand. Such an embodiment may utilize a first affinity ligand that comprises one member of a specific binding pair. A solid support is then suitably provided, which has the other member of the pair coupled to its surface. Non-limiting examples of specific binding pairs for use in this manner are covalently introduced groups, such as biotin covalently coupled to the first affinity ligand and avidin or streptavidin coupled to the solid support; and genetically introduced sequences, such as a hexahistidyl (His₆) sequence for interaction with the solid support through immobilized metal ion affinity chromatography (IMAC) principles of attachment (see e g Nilsson et al, Prot Exp Purif 11:1-16 (1997)). For example, the solid support may then consist of a dip stick, to which tertiary complexes formed may be immobilized through interaction between the first affinity ligand and the surface of the solid support. Thus, any species bound to the first affinity ligand may be "fished out" of solution and detected.

Another step in the method of the invention is the application of sample in such a way that binding of a target molecule, if present in the sample, to the first affinity ligand is enabled. Thus, the sample is applied to the immobilized first affinity ligand, and then incubated at the time, temperature and other conditions necessary for binding between affinity ligand and target molecule to occur. After such incubation, excess sample is preferably removed, e g by washing with a buffer solution. Through such removal, target molecules not bound to the first affinity ligand are removed.

After binding of target molecule to first affinity ligand has been enabled in the above manner, a second affinity ligand with affinity for the target molecule is applied under circumstances that enable binding of the second affinity ligand to the target molecule. The binding preferably occurs in a way that is non-competitive with respect to the first affinity ligand. The application of the second affinity ligand makes possible the formation of tertiary complexes comprising the first affinity ligand, the target molecule and the second affinity ligand. This will occur only if target molecules are present in the sample. Furthermore, it is likely that not all target molecules in the sample will be bound, the quota of bound target molecules to unbound target molecules depending on the reaction equilibria of the target molecule's binding to the two affinity ligands.

The determination whether target molecules are present in the sample or not depend on the detection of the tertiary complexes formed as described above. In order for this detection to yield the correct results, any species of the assay that are not bound to the immobilized first affinity ligand should be removed prior to detection. In this sense, a species is considered as bound to the first ligand whether it is directly bound, in the manner of the target molecule, or indirectly bound, in the manner of the second affinity ligand. In particular, the method of the invention comprises removing such second affinity ligands that have not bound to target molecules, since the presence of these non-bound second affinity ligands will yield false positive signals during subsequent detection, if not removed.

The sandwich assay method of the invention further comprises detection of the presence of the second affinity ligand, such presence being an indicator of the presence of a target molecule in the sample, since any remaining second affinity ligand is, or was at the time of removal described above, bound to a target molecule. The detection of the second affinity ligand may be performed through any method known in the art. For example, the second affinity ligand may for example be covalently coupled to a fluorescent marker molecule, to a radiolabel or to an active enzyme the presence of which may be ascertained through addition of substrate. Such coupling is suitably performed prior to use of the second affinity ligand in the method of the invention. Other means for detection useful in this step of the method of the present invention are sensors, e g for mass detection, which sensors may be based on optical or piezo-electrical detection principles; immunoelectromicroscopy; or the use of colloidal gold particles for visualization. Alternatively, the second affinity ligand may be detected by a third affinity ligand having affinity for the second affinity ligand. This third ligand, in turn, may then for example be coupled to a known marker or label. Various other detection means and techniques suitable for adaptation in the method of the present invention are known in the field of known immunoassays, and no undue experimentation is needed for such adaptation by the skilled person. In the case of an assay method according to the invention which is performed in standard microtiter plates, it is preferred to use a biotin-labeled second affinity ligand, which is detectable through a streptavidin-enzyme conjugate.

A feature of the method of the present invention is that at least one of the first and second affinity ligands is an affinity ligand other than an antibody. This ameliorates the problem of cross-linking between the first and second affinity ligands when there is no target present. In this way, e g the interference effects of HAIA in human samples can be reduced. In an embodiment of the method of the invention, an antibody against the target molecule is used as either first or second affinity ligand. Alternatively, both the first and second affinity ligands may be ligands other than antibodies.

In the context of the invention, an affinity ligand other than an antibody is engineered, using a naturally occurring protein, or domain(s) thereof, as a scaffold (i e starting-point structure). Protein engineering may for example be used to obtain libraries of protein variants, from which libraries suitable affinity ligands are subsequently selected. Such libraries may be constructed by combinatorial means, making possible the evolution of highly specific affinity ligands through randomization of a given number of amino acid residues in a scaffold protein. Suitable procedures for isolation of affinity ligands from combinatorial libraries are e g phage display technologies and other systems for *in vivo* or *in vitro* selection. These are known to the person of skill in the art, and examples are described in Fitzgerald, Drug Discovery Today 5:253-258 (2000).

Naturally occurring proteins, or domains thereof, for use as affinity ligands in the invention may for example be bacterial receptins or domains thereof. Particularly preferred bacterial receptins are staphylococcal protein A, streptococcal protein G and *Peptostreptococcus magnus* protein L, or domains thereof.

Engineered proteins for use as affinity ligands in the invention may be constructed using as scaffold a protein domain selected from the group consisting of domains of bacterial receptins, fibronectins, protease inhibitors, retinol binding proteins, bilin binding proteins, amylase inhibitors, CTLA-4, cytochromes and cellulose binding proteins. As examples of preferred scaffolds, domains from bacterial receptins are mentioned. Of such domains, domains derived from the group consisting of staphylococcal protein A, streptococcal protein G and *Peptostreptococcus magnus* protein L are especially preferred.

Thus, in an embodiment, an affinity ligand for use in the method of the present invention is constructed using any immunoglobulin binding domain of staphylococcal protein A as scaffold. Preferably, this domain is the B domain of staphylococcal protein A, or the Z domain derived therefrom (affinity ligands derived from the Z domain are sometimes referred to as Affibody® molecules in the present text, as well as in the literature). In another embodiment, any immunoglobulin binding domain of *Peptostreptococcus magnus* protein L is used as scaffold. In yet another embodiment, any immunoglobulin binding domain of streptococcal protein G is used as scaffold. In yet another embodiment, an albumin binding domain of streptococcal protein G is used as scaffold.

As alternatives to the proteins and protein domains mentioned above, affinity ligands may also be selected from a library of linear peptide variants, or from a library of cyclic peptide variants.

Above, the method of the invention has been set out in its most straightforward embodiment, utilizing only one species as first affinity ligand and one species as second affinity ligand. However, the method may be performed using any number of different species as capture or detector ligands. As an example, two different, detectable antibodies with different complementary determining regions may be used in combination as the "second affinity ligand" for the detection of bound target molecules, in order for example to enhance the sensitivity of the assay. Another example is the use of two non-antibody ligands of the same or different class with different binding specificities in an analogous manner.

To further explain and illustrate the invention, reference is now made to Fig 1. Fig 1A and 1B illustrate the problem of cross-linking by HAIA in the analysis of a human sample; a signal will be obtained through detection of the detection antibody, regardless of whether the target molecule is (Fig 1A) or is not (Fig 1B) present. Fig 1C illustrates the use of a non-antibody ligand as the second, "detector", affinity ligand. If there is no target molecule available, the non-antibody affinity ligand will not be cross-linked by the HAIA antibody (dotted line). Fig 1D is an illustration of another embodiment of the invention, in which a non-antibody affinity ligand is used as the first affinity ligand, and an antibody as the second affinity ligand. Fig 1E illustrates yet another embodiment of the invention, in which one type of non-antibody affinity ligand is used as both first and second affinity ligand in the method of the invention. Fig 1F illustrates yet another embodiment, in which two different types of non-antibody affinity ligands are used as first and second affinity ligands.

The invention will now be exemplified and further illustrated through the recital of experiments conducted in accordance therewith. The examples are not to be construed as limiting the scope of the invention as claimed.

### Example

### Materials and methods

### Affibody® molecules:

The selection by phage display and characterization of the Affibody® molecules used in this study has been described earlier. Briefly, using human myeloma IgA, recombinant human apolipoprotein A-1_{Milano} or recombinant respiratory syncytial virus (RSV) surface protein G targets, the Z_{IgA1} (Rönnmark *et al*, submitted manuscript (2002)), Z_{Apolipo24:4} (Nord et al, Nat Biotechnol 15:772 (1997)) and Z_{RSV1} (Hansson *et al*, Immunotechnology 4:237 (1999)) Affibody® molecules were selected by phage display technology from protein libraries constructed through randomization of thirteen surface-located positions in a 6 kDa domain (domain Z), derived from staphylococcal protein A (Nord et al, Protein Eng 8:601 (1995), Nord *et al, supra* (1997)). All three Affibody® molecules show approximately micromolar dissociation constants (K_{D}) for their respective targets. Z_{IgA1} binds both human IgA₁ and IgA₂ (Rönnmark *et al*, *supra).* In this study, Z_{RSV1}-ABD, (Z_{IgA1})₂-His₆ or (Z_{Apolipo24:4})₂-cys constructs produced in *Escherichia coli* were used, corresponding to monovalent or divalent constructs genetically fused to either a serum albumin binding domain (ABD), a hexahistidyl tag or a C-terminally positioned cysteine residue. Proteins were purified from shake flask cultures by HSA-affinity chromatography (ABD fusions), immobilized metal ion-affinity chromatography (His₆-fusions) or ion-exchange/reversed phase chromatography (cys fusions). When used as detection reagents, i e second affinity ligand, Affibody® molecules were biotinylated using a 25-fold molar excess of EZ-Link™ Sulfo-NHS-LC-Biotin NHS-Biotin (Pierce, Rockford, IL). Excess biotinylation reagent was removed by gel filtration using a PD10 column (Amersham Biosciences, Uppsala, Sweden). The Z constructs used in the study, including tags added to facilitate purification, are referred to as Z_{RSV}, Z_{IgA} and Z_{Apo} throughout this text (Table 1).

### Antibody reagents:

Non-biotinylated as well as biotinylated, affinity purified goat pAb specific for human IgA were used for IgA measurement (Sigma, St Louis, MO). For detection of human apolipoprotein A-1, non-biotinylated and biotinylated mouse mAb44 (IgG2b) and mAb110 (IgG1) were used (Mabtech, Nacka, Sweden). Other antibodies used to assess false positive signals caused by human serum antibodies were: goat pAb anti-mouse IgG + IgM (Jackson Immuno Research Laboratories Inc, West Grove, PA); biotinylated goat pAb anti-rabbit IgG (Vector, Burlingame, CA); mouse mAb 1-D1K (IgG1) specific for human interferon-gamma; biotinylated mouse mAb 12.1 (IgG1) specific for human interleukin-4; rat mAb TRFK5 (IgG1) specific for human interleukin-5 and biotinylated rat mAb 12G8 (IgG2a) specific for human interleukin-10 (Mabtech). All antibodies used are listed in Table 1.

### Standards and human serum samples:

Three different IgA standards were used, human polyclonal IgA1, human myeloma IgA2 (Calbiochem, San Diego, CA) and human polyclonal IgAl/IgA2 (Sigma) containing both IgA1 and IgA2. The apolipoprotein A-1 standard used was obtained from Sigma. For the assessment of IgA and apolipoprotein A-1 levels in human serum, a pool of more than 50 sera from healthy blood donors from Austria was used (PAA Laboratories GmbH, Linz, Austria).

### Sandwich assays for detection of human IgA:

ELISA plates (Costar 3590; Costar, Cambridge, MA) were incubated overnight at 4°C with phosphate-buffered saline (PBS) containing the capture reagent, i e the first affinity ligand. According to titrations made to yield high specific signals and low background, Affibody® molecules were used at 8 µg/ml, and pAb or mAb at 2 µg/ml. For all incubations, 100 µl of fluid per well was used. The wells were blocked with 200 µl of 1% bovine serum albumin (BSA) in PBS for 1 h at 20°C, and duplicates of serially diluted standard or dilutions of serum sample were added and incubated for 2 h at 20°C. All samples as well as subsequent detection reagents were diluted in PBS containing 0.1% BSA and 0.05% Tween. The wells were emptied and washed 5 times with PBS containing 0.05% Tween, and then incubated with biotinylated detection reagent at concentrations determined by titrations to yield a high signal-to-noise ratio; Affibody® and mAb reagents were used at 1 µg/ml and pAb reagents at 0.5 µg/ml. Following an incubation of 1 h at 20°C, the wells were washed and 50 ng/ml of streptavidin-alkaline phosphatase conjugate (Mabtech) was added. Finally, 100 µl of p-nitrophenyl phosphate (Sigma) buffer was added to each well and the plates were read at 405 nm (Molecular Device, Menlo Park, CA) after approximately 1 h. The level of background for each combination of capture and detection reagents was assessed using buffer only. The lower detection level of each system was determined as the lowest standard concentration giving an absorbance value two times the background level of the system. The concentration of analyte in a pool of human sera was quantified by analysis of diluted serum samples and subsequent comparison to a protein standard curve created with a four-parameter Rodbard function (SoftMaxPro; Molecular Device).

### Analysis of false positive signals caused by human serum antibodies in sandwich assays:

In principle, the same protocol as above was used. Affibody®, mAb or pAb were adsorbed to ELISA plates as above, and after blocking and washing, consecutive fivefold dilutions of a human serum pool were added and incubated. Biotinylated Affibody®, mAb or pAb displaying non-matched specificities were subsequently used to assess the level of false positive signals. The absorbance signal caused by human serum antibodies was estimated by subtracting the background absorbance of the system (buffer only) from the absorbance obtained with the serum samples. In order to compare results from different experiments, internal standards were included in each experiment.

| Table 1. Affibody^{®} molecules and antibodies used in the study | | | | |
|---|---|---|---|---|
| Reagent | Origin | Specificity | Label | Designation |
| Affibody^{®} | bacteria | human IgA | - | Z_{IgA} |
| | bacteria | human IgA | biotin | Z_{IgA}-b |
| | bacteria | human apolipoprotein A-1 | - | Z_{Apo} |
| | bacteria | human apolipoprotein A-1 | biotin | Z_{Apo}-b |
| | bacteria | RSV | - | Z_{RSV} |
| PAb | goat | human IgA | - | pAb_{IgA} |
| | goat | human IgA | biotin | pAb_{IgA}-b |
| | goat | mouse IgG+IgM | - | pAb_{mIg} |
| | goat | rabbit IgG | biotin | pAb_{RIgG}-b |
| MAb | mouse | human apolipoprotein A-1 | - | mAb110_{Apo} |
| | mouse | human apolipoprotein A-1 | biotin | mAb110_{Apo}-b |
| | mouse | human apolipoprotein A-1 | - | mAb44_{Apo} |
| | mouse | human apolipoprotein A-1 | biotin | mAb44_{Apo}-b |
| | mouse | human interferon-gamma | - | mmAb_{IFN} |
| | mouse | human interleukin-4 | biotin | mmAb_{IL-4}-b |
| | rat | human interleukin-5 | - | rmAb_{IL-5} |
| | rat | human interleukin-10 | biotin | rmAb_{IL-10} -b |

### Results

### Analysis of IgA standards and human serum IgA levels using a sandwich assay based on polyclonal antibodies and/or Affibody^{®} molecules:

An Affibody^{®} capable of selective recognition of human IgA was used as an alternative to antibody reagents in accordance with the invention. The Affibody^{®} Z_{IgA} was previously selected from a protein library constructed by combinatorial engineering of a single staphylococcal protein A domain, and shown to bind human IgA1 and IgA2 with equal affinity (Rönnmark et al, *supra).* Criss-cross capture and detection combinations of Z_{IgA} and IgA-specific goat pAb (pAb_{IgA}) were analyzed for reactivity with the IgA1, IgA2 and IgA1/IgA2 standards. As a comparative example using a known method, the ELISA format using pAb for capture as well as detection (pAb_{IgA}/pAb_{IgA}-b) displayed a comparable reactivity with the different standards (Fig 2A) and a lower detection limit of the IgA standards between 62 and 125 pg/ml (Table 2). Interestingly, the combination of Z_{IgA} and pAb_{IgA}-b according to the invention was fully comparable both in terms of reactivity with the different standards (Fig 2B) and the lower detection limits (Table 2). The reverse combination (pAb_{IgA}/Z_{IgA}-b) displayed a lower reactivity with all three standards, and even less sensitive was detection based on Z_{IgA}/Z_{IgA}-b (Fig 2C & D). For these latter formats, the lowest concentrations of IgA standards detectable were 4 and 200-800 ng/ml, respectively (Table 2).

The different systems were subsequently used to quantify IgA in a pool of human sera using serum dilutions adjusted according to the detection range of each system. Analysis of serum diluted 3 x 10⁶ times using pAb_{IgA}/pAb_{IgA}-b (comparative example) and Z_{IgA}/pAb_{IgA}-b (according to the invention) yielded IgA levels of 2.7 and 3.0 mg/ml, respectively (Table 2). Determination using pAb_{IgA}/Z_{IgA}-b (according to the invention) resulted in an IgA concentration of 2.2 mg/ml using serum samples diluted 1 x 10⁴ or 1 x 10⁵ times for the analysis. Analysis with the less sensitive format Z_{IgA}/Z_{IgA}-b (according to the invention) using serum diluted 500 times gave an IgA. concentration of 4.8 mg/ml.

Taken together, these analyses showed that the non-immunoglobulin affinity ligand Z_{IgA} could be used in conjunction with a pAb reagent in a sandwich assay method for specific target quantification and that the use of this Affibody^{®} as capture reagent, i e first affinity ligand, was the preferable format.

| Table 2. Comparison of human IgA sandwich assays based on an Affibody^{®} (Z_{IgA}) and/or polyclonal antibodies (pAb_{IgA}) | | | | | |
|---|---|---|---|---|---|
| Assay system | | Lower detection limit for IgA standard^{a} | | | Determination of serum IgA^{b} |
| Coating | Detection | IgA1/Ig A2 (ng/ml) | IgA1 (ng/ml) | IgA2 (ng/ml) | (mg/ml) |
| pAb_{IgA} | pAb_{IgA}-b | 0.062 | 0.062 | 0.12 | 2.7 |
| Z_{IgA} | pAb_{IgA}-b | 0.062 | 0.062 | 0.12 | 3.0 |
| pAb_{IgA} | Z_{IgA}-b | 4.0 | 4.0 | 4.0 | 2.2 |
| Z_{IgA} | Z_{IgA}-b | 800 | 200 | 400 | 4.8 |

| | | | | | |
|---|---|---|---|---|---|
| ^{a} Serial two-fold dilutions of IgA standards were analyzed in each system. The lower detection limit was defined as the lowest concentration giving an absorbance twice the background of each system. ^{b} A serum pool was analyzed at dilutions adjusted according to the sensitivity of each system and the IgA levels quantified by comparison to an IgA1/IgA2 standard. The serum IgA concentration was determined by multiplying the IgA level with the dilution factor. | | | | | |

### False positive signals caused by human serum antibodies in sandwich assay methods:

According to the invention, the bacterial origin of the Z_{IgA} affinity reagent addresses the problem of false positive signals associated with human serum antibodies in capture immunoassays, through the use of Affibody^{®}/antibody reagent combinations in a sandwich assay method. By combining reagents displaying non-matched specificities, the signals observed with each system after incubation with serum could be linked to the presence of cross-linking antibodies. All combinations of two antibodies (goat pAb, mouse mAb and/or rat mAb) resulted in different levels of cross-linking, in some cases also after diluting the serum pool 3,125 times (Fig 3A). In contrast, none of the combinations of antibodies for capture and Affibody^{®} molecules for detection, or vice versa, were cross-linked (Fig 3B). Noteworthy, although although antibody reactivity with non-modified framework regions of the S. *aureus* protein A-derived Affibody^{®} molecules could be expected as a result of natural exposure to bacteria, the combination of two Affibody^{®} molecules displaying different specificities (Z_{RSV} and Z_{Apo}-b) were not cross-linked by human sera (Fig 3B).

### Measurement of human apolipoprotein A-1 using a sandwich assay method based on Affibody^{®} and/or monoclonal antibodies:

A second assay system based on Affibody^{®} and mAb was evaluated (Fig 4). In this case, the analyte, a non-Ig molecule (apolipoprotein A-1) was measured using the Affibody^{®} Z_{Apo} in combination with specific mAbs. When comparing the ability of combinations of Z_{Apo}, mAb110_{Apo} and mAb44_{Apo} to detect apolipoprotein A-1 standard, mAb110_{Apo} and mAb44_{Apo}-b displayed the highest sensitivity. The combinations using Z_{Apo} for capture and either mAb for detection also detected apolipoprotein A-1, albeit with lower sensitivity. All functional combinations of reagents were used to quantify apolipoprotein A-1 in a pool of human sera diluted according to the sensitivity of each system (Table 3). Appropriate absorbance signals in each system were obtained using serum diluted 1-3 x 10⁶ times for mAb110_{Apo}/mAb44_{Apo}-b, 1-3 x 10⁵ times for the other systems. The serum concentrations of apolipoprotein A-1 determined were 0.8-1.7 mg/ml using different combinations of Z_{Apo} and/or mAbs.

| Table 3. Comparison of human apolipoprotein A-1 sandwich assay systems based on an Affibody^{®} (Z_{Apo}) and/or monoclonal antibodies (mAb44_{Apo} and mAb110_{Apo}) | | | |
|---|---|---|---|
| Assay system | | Lower detection limit of apolipoprotein A-1 standard^{a} | Serum levels of apolipo-protein A-1^{b} |
| Coating | Detection | ng/ml | mg/ml |
| mAb110_{Apo} | mAb44_{Apo}-b | 0.062 | 0.8 |
| mAb44_{Apo} | mAb110_{Apo}-b | 2.0 | 1.3 |
| Z_{Apo} | mAb110_{Apo}-b | 0.49 | 1.6 |
| Z_{Apo} | mAb44_{Apo}-b | 0.49 | 1.7 |
| Z_{Apo} | mAb44_{Apo}-b & | 0.49 | 1.7 |
| | mAb110_{Apo}-b | | |

| | | | |
|---|---|---|---|
| ^{a} Serial two-fold dilutions of apolipoprotein A-1 standard were analyzed in each system. The lower detection limit was defined as the lowest concentration giving an absorbance twice the background of each system. ^{b} A serum pool was analyzed at dilutions adjusted according to the sensitivity of each system and the apolipoprotein A-1 levels quantified by comparison to a standard. The serum apolipoprotein A-1 concentration was determined by multiplying the determined levels with the dilution factor. | | | |

## Claims

1. Sandwich assay method for detecting the presence of a target molecule in a human sample comprising a complex biological fluid containing heterophilic anti-animal Ig antibodies, which assay comprises:
- providing a first affinity ligand with affinity for the target molecule, which affinity ligand is capable of being immobilized to a solid support;
- applying the sample in such a way that binding of a target molecule, if present in the sample, to the first affinity ligand is enabled;
- applying a second affinity ligand with affinity for the target molecule, the application enabling binding of the second affinity ligand to the target molecule;
- removing second affinity ligand not bound to target molecule; and
- detecting the presence of the second affinity ligand, such presence being an indicator of the presence of a target molecule in the sample;
the first affinity ligand being immobilized to the solid support at any stage before said detection,
in which method
at least one of the first and second affinity ligands is an affinity ligand other than an antibody, said at least one affinity ligand being an engineered protein selected from a library constructed by combinatorial means through randomization of a given number of amino acids in a scaffold protein consisting of a naturally occurring protein or domain(s) thereof, and
said first and second affinity ligands are different from each other in terms of structure or origin.

2. Sandwich assay method according to claim 1, in which the first affinity ligand is provided immobilized to the solid support.

3. Sandwich assay method according to claim 1, in which the first affinity ligand is immobilized to the solid support during performance of the method.

4. Sandwich assay method according to any one of claims 2 and 3, in which the solid support is selected from microtiter plates; compact discs comprising microfluidic channels; protein array chips; membranes; microparticles; pin structures; stick structures; sensor surfaces; and cell surfaces.

5. Sandwich assay method according to claim 4, in which the solid support is a microtiter plate.

6. Sandwich assay method according to any one of the preceding claims, which further comprises removing target molecules not bound to the first affinity ligand.

7. Sandwich assay method according to any one of the preceding claims, in which the second affinity ligand is an affinity ligand other than an antibody.

8. Sandwich assay method according to any one of the preceding claims, in which the first affinity ligand is an affinity ligand other than an antibody.

9. Sandwich assay method according to any one of the preceding claims, in which both the first and the second affinity ligand is an affinity ligand other than an antibody.

10. Sandwich assay method according to any one of the preceding claims, in which at least one of the first and second affinity ligands is an engineered protein, constructed by using as scaffold a protein domain selected from domains of bacterial receptins; fibronectins; protease inhibitors; retinol binding proteins; bilin binding proteins; amylase inhibitors; CTLA-4; cytochromes; and cellulose binding proteins.

11. Sandwich assay method according to claim 10, in which the scaffold is selected from bacterial receptin domains.

12. Sandwich assay method according to claim 11, in which the scaffold is selected from the immunoglobulin binding domains of staphylococcal protein A.

13. Sandwich assay method according to claim 12, in which the scaffold is the B domain of staphylococcal protein A.

14. Sandwich assay method according to claim 13, in which the scaffold is the Z domain derived from the B domain of staphylococcal protein A.

15. Sandwich assay method according to claim 11, in which the scaffold is selected from the immunoglobulin binding domains of *Peptostreptococcus magnus* protein L.

16. Sandwich assay method according to claim 11, in which the scaffold is selected from the immunoglobulin binding domains of streptococcal protein G.

17. Sandwich assay method according to claim 11, in which the scaffold is selected from the albumin binding domains of streptococcal protein G.

18. Sandwich assay method according to any one of the preceding claims, in which the library is constructed using phage display technology.

19. Sandwich assay method according to any one of the preceding claims, in which the complex biological fluid is selected from serum, plasma, saliva, whole blood, plasma from plasmapheresis, cerebrospinal fluid, amniotic fluid, urine, semen, cord blood, supernatants from cell culture, cell culture media, exsudate and aspirate.

20. Sandwich assay method according to any one of the preceding claims, in which the sample is a human serum sample.

## Patentansprüche

1. Sandwich-Assay-Verfahren zum Nachweisen des Vorhandenseins eines Zielmoleküls in einer Probe von einem Menschen, die eine komplexe biologische Flüssigkeit umfasst, die heterophile Anti-Tier-Ig-Antikörper enthält, wobei der Assay Folgendes umfasst:
- Bereitstellen eines ersten Affinitätsliganden mit Affinität für das Zielmolekül, wobei der Affinitätsligand auf einem festen Träger immobilisiert werden kann;
- Aufbringen der Probe derart, dass eine Bindung eines Zielmoleküls, falls in der Probe vorhanden, an den ersten Affinitätsliganden ermöglicht wird;
- Aufbringen eines zweiten Affinitätsliganden mit Affinität für das Zielmolekül, wobei das Aufbringen eine Bindung des zweiten Affinitätsliganden an das Zielmolekül ermöglicht;
- Entfernen des zweiten Affinitätsliganden, der nicht an das Zielmolekül gebunden ist; und
- Nachweisen des Vorhandenseins des zweiten Affinitätsliganden, wobei ein derartiges Vorhandensein ein Indikator des Vorhandenseins eines Zielmoleküls in der Probe ist;
wobei der erste Affinitätsligand in einer beliebigen Phase vor dem Nachweis auf dem festen Träger immobilisiert wird,
wobei in diesem Verfahren
mindestens einer des ersten und des zweiten Affinitätsliganden ein Affinitätsligand ist, der kein Antikörper ist, wobei der mindestens eine Affinitätsligand ein gentechnisch hergestelltes Protein ist, das aus einer Bibliothek ausgewählt ist, die mit einem kombinatorischen Mittel durch Randomisierung einer gegebenen Anzahl von Aminosäuren in einem Gerüstprotein, das aus einem natürlich vorkommenden Protein oder einer oder mehreren Domänen davon besteht, konstruiert wurde, und
wobei der erste und der zweite Affinitätsligand sich in Bezug auf ihre Struktur oder ihren Ursprung voneinander unterscheiden.

2. Sandwich-Assay-Verfahren nach Anspruch 1, wobei der erste Affinitätsligand auf dem festen Träger immobilisiert bereitgestellt wird.

3. Sandwich-Assay-Verfahren nach Anspruch 1, wobei der erste Affinitätsligand während der Durchführung des Verfahrens auf dem festen Träger immobilisiert wird.

4. Sandwich-Assay-Verfahren nach einem der Ansprüche 2 und 3, wobei der feste Träger aus Mikrotiterplatten; Compact-Disks, die mikrofluidische Kanäle umfassen; Proteinarray-Chips; Membranen; Mikroteilchen; Stiftstrukturen; Stabstrukturen; Sensoroberflächen und Zelloberflächen ausgewählt ist.

5. Sandwich-Assay-Verfahren nach Anspruch 4, wobei der feste Träger eine Mikrotiterplatte ist.

6. Sandwich-Assay-Verfahren nach einem der vorhergehenden Ansprüche, das weiterhin das Entfernen der Zielmoleküle, die nicht an den ersten Affinitätsliganden gebunden sind, umfasst.

7. Sandwich-Assay-Verfahren nach einem der vorhergehenden Ansprüche, wobei der zweite Affinitätsligand ein Affinitätsligand ist, der kein Antikörper ist.

8. Sandwich-Assay-Verfahren nach einem der vorhergehenden Ansprüche, wobei der erste Affinitätsligand ein Affinitätsligand ist, der kein Antikörper ist.

9. Sandwich-Assay-Verfahren nach einem der vorhergehenden Ansprüche, wobei sowohl der erste als auch der zweite Affinitätsligand Affinitätsliganden sind, die keine Antikörper sind.

10. Sandwich-Assay-Verfahren nach einem der vorhergehenden Ansprüche, wobei mindestens einer des ersten und des zweiten Affinitätsliganden ein gentechnisch hergestelltes Protein ist, das durch Verwendung einer Proteindomäne als Gerüst konstruiert wurde, die aus Domänen von bakteriellen Receptinen; Fibronektinen; Proteasehemmern; Retinol-Bindeproteinen; Bilin-Bindeproteinen; Amylasehemmern; CTLA-4; Cytochromen und Cellulose-Bindeproteinen ausgewählt sind.

11. Sandwich-Assay-Verfahren nach Anspruch 10, wobei das Gerüst aus bakteriellen Rezeptin-Domänen ausgewählt ist.

12. Sandwich-Assay-Verfahren nach Anspruch 11, wobei das Gerüst aus den Immunglobulin-Bindedomänen von Staphylokokken-Protein A ausgewählt ist.

13. Sandwich-Assay-Verfahren nach Anspruch 12, wobei das Gerüst die B-Domäne von Staphylokokken-Protein A ist.

14. Sandwich-Assay-Verfahren nach Anspruch 13, wobei das Gerüst die Z-Domäne ist, die von der B-Domäne von Staphylokokken-Protein A abgeleitet ist.

15. Sandwich-Assay-Verfahren nach Anspruch 11, wobei das Gerüst aus den Immunglobulin-Bindedomänen von *Peptostreptococcus-magnus-Protein* L ausgewählt ist.

16. Sandwich-Assay-Verfahren nach Anspruch 11, wobei das Gerüst aus den Immunglobulin-Bindedomänen von Streptokokken-Protein G ausgewählt ist.

17. Sandwich-Assay-Verfahren nach Anspruch 11, wobei das Gerüst aus den Albumin-Bindedomänen von Streptokokken-Protein G ausgewählt ist.

18. Sandwich-Assay-Verfahren nach einem der vorhergehenden Ansprüche, wobei die Bibliothek unter Verwendung von Phagendisplay-Technologie konstruiert wird.

19. Sandwich-Assay-Verfahren nach einem der vorhergehenden Ansprüche, wobei die komplexe biologische Flüssigkeit aus Serum, Plasma, Speichel, Vollblut, Plasma aus einer Plasmapherese, Gehirn-Rückenmarksflüssigkeit, Fruchtwasser, Urin, Samenflüssigkeit, Nabelschnurblut, Überständen von einer Zellkultur, Zellkulturmedium, Exsudat und Aspirat ausgewählt ist.

20. Sandwich-Assay-Verfahren nach einem der vorhergehenden Ansprüche, wobei die Probe eine Serumprobe von einem Menschen ist.

## Revendications

1. Procédé de dosage en sandwich pour la détection de la présence d'une molécule cible dans un échantillon d'éléments du corps humain comprenant un liquide biologique complexe contenant des anticorps hétérophiles anti-Ig animale, lequel dosage comprend :
- la fourniture d'un premier ligand pour dosage par affinité ayant une affinité pour la molécule cible, lequel ligand pour dosage par affinité étant capable d'être immobilisé sur un support solide ;
- l'application de l'échantillon d'une manière telle que la liaison d'une molécule cible, si elle est présente dans l'échantillon, au premier ligand pour dosage par affinité est permise ;
- l'application d'un second ligand pour dosage par affinité ayant une affinité pour la molécule cible, l'application permettant la liaison du second ligand pour dosage par affinité à la molécule cible ;
- l'élimination du second ligand pour dosage par affinité non lié à la molécule cible ; et
- la détection de la présence d'un second ligand pour dosage par affinité, laquelle présence étant un indicateur de la présence d'une molécule cible dans l'échantillon ;
le premier ligand pour dosage par affinité étant immobilisé au support solide à n'importe quel stade avant ladite détection,
dans lequel procédé
au moins l'un des premier et second ligands pour dosage par affinité est un ligand pour dosage par affinité autre qu'un anticorps, ledit au moins un ligand pour dosage par affinité étant une protéine génétiquement conçue choisie parmi une bibliothèque construite par des moyens combinatoires d'une manière aléatoire d'un nombre donné d'acides aminés dans une protéine d'échafaudage constituée d'une protéine d'origine naturelle ou d'un (de) domaine(s) de celle-ci, et
lesdits premier et second ligands pour dosage par affinité étant différents l'un de l'autre en termes de structure ou d'origine.

2. Procédé de dosage en sandwich selon la revendication 1, dans lequel le premier ligand pour dosage par affinité est proposé immobilisé au support solide.

3. Procédé de dosage en sandwich selon la revendication 1, dans lequel le premier ligand pour dosage par affinité est immobilisé au support solide durant l'exécution du procédé.

4. Procédé de dosage en sandwich selon l'une quelconque des revendications 2 et 3, dans lequel le support solide est choisi parmi les plaques de microtitrage ; les disques compacts comprenant des canaux microfluidiques ; les puces de type réseau de protéines ; les membranes ; les microparticules ; les structures en aiguille ; les structures en baguette ; les surfaces capteurs ; et les surfaces cellulaires.

5. Procédé de dosage en sandwich selon la revendication 4, dans lequel le support solide est une plaque de microtitrage.

6. Procédé de dosage en sandwich selon l'une quelconque des revendications précédentes, qui comprend en outre l'élimination des molécules cibles non liées au premier ligand pour dosage par affinité.

7. Procédé de dosage en sandwich selon l'une quelconque des revendications précédentes, dans lequel le second ligand pour dosage par affinité est un ligand pour dosage par affinité autre qu'un anticorps.

8. Procédé de dosage en sandwich selon l'une quelconque des revendications précédentes, dans lequel le premier ligand pour dosage par affinité est un ligand pour dosage par affinité autre qu'un anticorps.

9. Procédé de dosage en sandwich selon l'une quelconque des revendications précédentes, dans lequel à la fois le premier et le second ligands pour dosage par affinité est un ligand pour dosage par affinité autre qu'un anticorps.

10. Procédé de dosage en sandwich selon l'une quelconque des revendications précédentes, dans lequel au moins l'un des premier et second ligands pour dosage par affinité est une protéine génétiquement conçue, construite en utilisant un échafaudage d'un domaine protéique choisie parmi les domaines des réceptines bactériennes ; des fibronectines ; des inhibiteurs de protéase ; des protéines de liaison au rétinol ; des protéines de liaison à la biline ; des inhibiteurs de l'amylase ; de CTLA-4 ; des cytochromes ; et des protéines de liaison à la cellulose.

11. Procédé de dosage en sandwich selon la revendication 10, dans lequel l'échafaudage est choisi parmi les domaines des réceptines bactériennes.

12. Procédé de dosage en sandwich selon la revendication 11, dans lequel l'échafaudage est choisi parmi les domaines de liaison à l'immunoglobuline de la protéine staphylococcique A.

13. Procédé de dosage en sandwich selon la revendication 12, dans lequel l'échafaudage est le domaine B de la protéine staphylococcique A.

14. Procédé de dosage en sandwich selon la revendication 13, dans lequel l'échafaudage est le domaine Z dérivé du domaine B de la protéine staphylococcique A.

15. Procédé de dosage en sandwich selon la revendication 11, dans lequel l'échafaudage est choisi parmi les domaines de liaison à l'immunoglobuline de la protéine L de *Peptostreptococcus magnus.*

16. Procédé de dosage en sandwich selon la revendication 11, dans lequel l'échafaudage est choisi parmi les domaines de liaison à l'immunoglobuline de la protéine streptococcique G.

17. Procédé de dosage en sandwich selon la revendication 11, dans lequel l'échafaudage est choisi parmi les domaines de liaison à l'albumine de la protéine streptococcique G.

18. Procédé de dosage en sandwich selon l'une quelconque des revendications précédentes, dans lequel la bibliothèque est construite en utilisant la technologie reposant sur l'affichage à la surface des phages.

19. Procédé de dosage en sandwich selon l'une quelconque des revendications précédentes, dans lequel le liquide biologique complexe est choisi parmi le sérum, le plasma, la salive, le sang entier, le plasma provenant de la plasmaphérèse, le liquide cérébro-spinal, le liquide amniotique, l'urine, le sperme, le sang du cordon ombilical, les surnageants de cultures cellulaires, le milieu de culture cellulaire, l'exsudat et l'aspirat.

20. Procédé de dosage en sandwich selon l'une quelconque des revendications précédentes, dans lequel l'échantillon est un échantillon de sérum humain.
